(19) Europäisches Patentamt
European Patent Office
Office européen des brevets

(11) Veröffentlichungsnummer: **0 146 064**
**A2**

(12) # EUROPÄISCHE PATENTANMELDUNG

(21) Anmeldenummer: **84114626.9**

(22) Anmeldetag: **25.05.82**

(51) Int. Cl.⁴: **C 07 D 249/08**, A 01 N 43/653

(30) Priorität: **01.06.81 DE 3121676**

(43) Veröffentlichungstag der Anmeldung: **26.06.85 Patentblatt 85/26**

(84) Benannte Vertragsstaaten: **AT BE CH DE FR GB IT LI NL SE**

(60) Veröffentlichungsnummer der früheren Anmeldung nach Art. 76 EPÜ: **0068144**

(71) Anmelder: **HOECHST AKTIENGESELLSCHAFT, Postfach 80 03 20, D-6230 Frankfurt am Main 80 (DE)**

(72) Erfinder: **Hertenstein, Ulrich, Dr., Goethestrasse 3, D-8901 Garblingen (DE)**
Erfinder: **Mildenberger, Hilmar, Dr., Fasanenstrasse 24, D-6233 Kelkheim (Taunus) (DE)**
Erfinder: **Sachse, Burkhard, Dr., An der Ziegelei 30, D-6233 Kelkheim (Taunus) (DE)**
Erfinder: **Hartz, Peter, Dr., An der Ziegelei 30, D-6233 Kelkheim (Taunus) (DE)**

(54) **1,1-Diphenyl-2-triazolyl-2-alkyl-ethane, ihre Herstellung und ihre Verwendung als Biozide.**

(57) Die neuen 1,1-Diphenyl-2-triazolyl-2-alkyl-ethane der allgemeinen Formel (I)

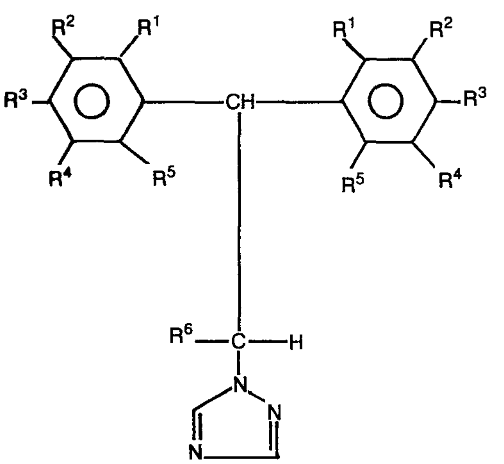

(I)

in der die Symbole folgende Bedeutung haben:
$R^1$ bis $R^5$ sind gleich oder verschieden H, Hal, $CF_3$, $(C_1-C_8)$Alkyl oder $(C_1-C_6)$Alkoxy, wobei auch die Substituenten mit gleichem Index in beiden Phenylresten jeweils verschieden sein können, und $R^6$ $(C_1-C_4)$Alkyl,
sowie ihre Salze, Komplexsalze und Quarternisierungsprodukte können aus den Verbindungen der allgemeinen Formel (II)

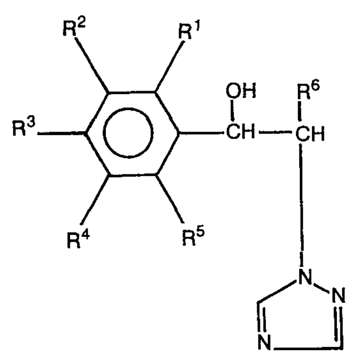

(II)

und einem Äquivalent eines Aromaten der allgemeinen Formel (III)



ACTORUM AG

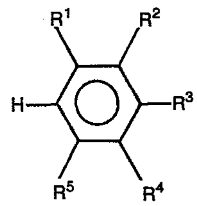

(III)

unter Friedel-Crafts-Bedingungen hergestellt werden. Diese Verbindungen sind als Biozide, insbesondere als Fungizide geeignet.

HOECHST AKTIENGESELLSCHAFT HOE 81/F 807 B Dr.IS
Teilanmeldung aus der Europäischen Patentanmeldung Nr. 82104542.4
(HOE 81/F 807)

# 1,1-Diphenyl-2-triazolyl-2-alkyl-ethane, ihre Herstellung und ihre Verwendung als Biozide

Die Erfindung betrifft neue 1,1-Diphenyl-2-triazolyl-2-alkyl-ethane, ein Verfahren zur Herstellung dieser Substanzklasse sowie ihre Verwendung als Biozide, insbesondere als Fungizide.

In der DE-A 28 21 971 werden bereits - dargestellt durch eine allgemeine Formel - Verbindungen aufgeführt, die als 1,1-Diphenyl-2-triazolyl-ethane aufgefaßt werden können, wobei jedoch diese substituierten Ethane in der 2-Stellung keine weiteren Substituenten tragen. Ein Beispiel für eine solche Verbindung (S. 12, Zeile 2 der DE-A) ist das 1-(2,4-Dichlorphenyl)-1-(4-chlorphenyl)-2-(1,2,4-triazol-1-yl)-ethan.

Auch in der EP-A 0 037 049 werden in 2-Stellung lediglich einen Triazolylsubstituenten tragende 1,1-Diphenyl-ethane beschrieben, bei denen im übrigen einer der beiden Phenylreste zwingend in p-Stellung durch einen weiteren Phenylrest substituiert ist.

Es wurde gefunden, daß bestimmte, bisher nicht beschriebene 1,1-Diphenyl-2-triazolyl-2-alkyl-ethane - verglichen mit den bereits bekannten Verbindungen - eine höhere fungizide Potenz, insbesondere gegen Rost und Mehltau, aufweisen und herbizid wirksam sein können.

Gegenstand der Erfindung sind daher die 1,1-Diphenyl-2-triazolyl-2-alkyl-ethane der allgemeinen Formel (I)

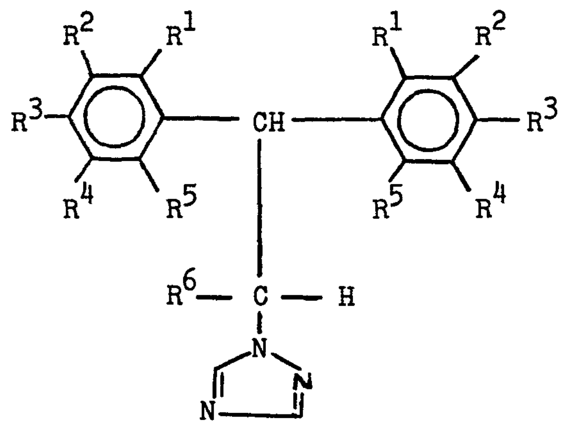

(I)

in der die Symbole folgende Bedeutung haben:

$R^1$ bis $R^5$ sind gleich oder verschieden H, Hal, $CF_3$, $(C_1-C_8)$Alkyl oder $(C_1-C_6)$Alkoxy, wobei auch die Substituenten mit gleichem Index in beiden Phenylresten jeweils verschiedene sein können, und

$R^6$ $(C_1-C_4)$Alkyl,

sowie ihre Salze, Komplexsalze und Quaternisierungsprodukte.

Von diesen Verbindungen sind die mit $R^6 = CH_3$ oder $C_2H_5$ bevorzugt, ganz besonders bevorzugt sind die mit $R^6 = CH_3$.

Diese neuen Verbindungen werden aus, eine alkoholische OH-Gruppe tragenden Verbindungen der Formel (II)

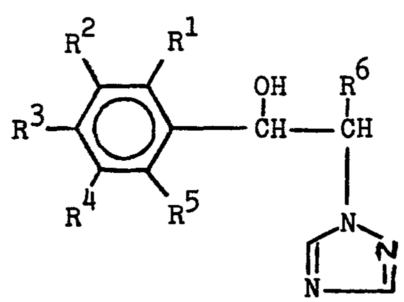

(II)

durch Reaktion mit Aromaten der Formel (III)

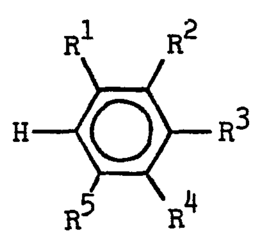

(III)

erhalten.

0146064

Man arbeitet im Temperaturbereich von -15 bis +150 °C, vorzugsweise von -10 bis 110 °C, in Gegenwart eines sauren Katalysators, gegebenenfalls bei Anwesenheit eines Verdünnungsmittels. Unter sauren Katalysatoren sind hierbei die üblichen Friedel-Crafts-Katalysatoren zu verstehen, wie sie - ebenso wie geeignete Verdünnungsmittel - z. B. im Houben-Weyl, Methoden der org. Chemie, Bd. 7/2a, S. 17 bis 21, beschrieben sind. Bevorzugt werden Tetrachlorethan und Aluminiumtrichlorid. Vom Katalysator setzt man 1,1 bis 2,5 Äquivalente, vom Arylkohlenwasserstoff der Formel (III) zumindest 1 Äquivalent, bezogen auf die Verbindung der Formel (II), ein.

Für die Herstellung der 1,1-Diphenyl-2-triazolyl-2-alkylethane geeignete Ausgangsverbindungen der Formel (II) sind beispielsweise:
1-(2,4-Dichlorphenyl)-1-hydroxy-2-(1,2,4-triazol-1-yl)-propan oder das 1-Pentamethylphenyl-1-hydroxy-2-(1,2,4-triazol-1-yl)-propan.

Unter Aromaten der allgemeinen Formel (III) werden z.B. verstanden:
Chlorbenzol, Brombenzol, 1,3-Dichlorbenzol, 1,2-Dichlorbenzol, 1,4-Dichlorbenzol, Toluol, Xylol, Mesitylen, 3-Chlortoluol, 4-Chlortoluol und Anisol.

Bei der Synthese geht man im einzelnen so vor, daß man die Verbindungen der Formel (II), den Arylkohlenwasserstoff der Formel (III) und gegebenenfalls ein Verdünnungsmittel vorlegt und den Katalysator bei ca. -10°C langsam zudosiert. Man läßt innerhalb 30 min auf Raumtemperatur kommen und heizt dann bis zur beginnenden Gasentwicklung auf. Ist diese beendet, so heizt man 30 min nach, läßt auf Raumtemperatur abkühlen, gießt auf Eis, macht mit 50 %iger NaOH unter Eiskühlung stark alkalisch und arbeitet dann die organische Phase wie üblich auf.

Der zu den erfindungsgemäßen Produkten führende Reaktionsverlauf sei nachstehend am Beispiel der Umsetzung von 1,3-Dichlorbenzol mit 1-(4-Chlorphenyl)-1-hydroxy-2-(1,2,4-triazol-1-yl)-propan aufgezeigt:

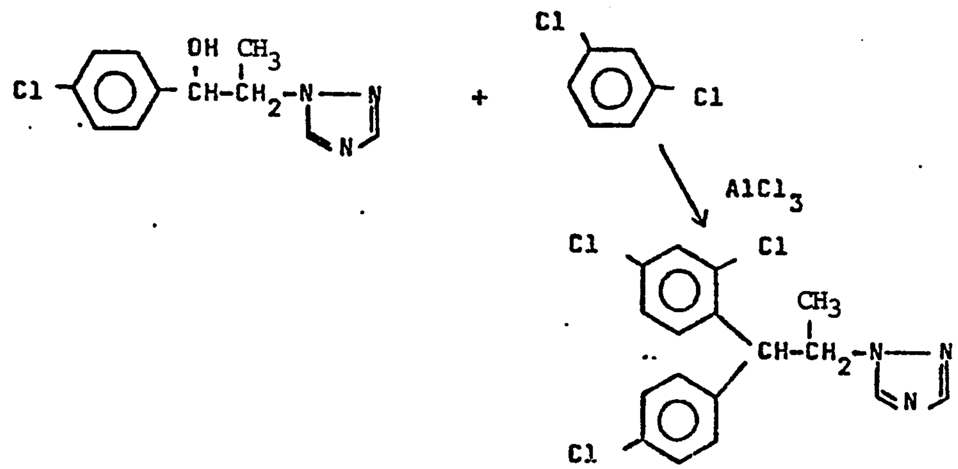


Die erfindungsgemäßen Verbindungen können in E- und Z-Form auftreten. Als basische Verbindungen sind sie ferner zur Bildung von Salzen, Komplexsalzen und Quaternisierungsprodukten befähigt. Genannt seien Salze organischer und anorganischer Säuren, wie z. B. Acetate, Fumarate, Oxalate, Benzoate, Nitrate, Bromide, Chloride, Sulfate, Salze der Naphthalinsulfonsäuren, Komplexe mit Metallen der Gruppe 1b, 2b, 4b, 8b des Periodensystems, etwa Kupfer, Zink und Zinn und Quaternisierungsprodukte mit Alkyl- und Phenacylhalogeniden. Die Herstellung solcher Verbindungen erfolgt nach den allgemein üblichen Methoden.

Die erfindungsgemäßen Verbindungen der Formel (I) zeichnen sich durch eine hervorragende fungizide Wirkung aus, insbesondere z.B. bei der Anwendung im Pflanzenschutz. Dabei lassen sich bereits in das pflanzliche Gewebe eingedrungene pilzliche Krankheitserreger erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft

werden können. Das Wirkungsspektrum der beanspruchten Verbindungen erfaßt z.B. neben verschiedenen Rostarten, Phytophthora infestans, Plasmopara viticola und Piricularia oryzae, vor allem aber Echte Mehltaupilze im Obst-, Gemüse- Getreide- und Zierpflanzenbau. Besonders hervorzuheben ist die ausgezeichnete Wirkung der Verbindungen gegen Mehltauarten, die gegen Benzimidazolderivate (z.B. Benomyl, Carbendazim) resistent sind.

Die Substanzen eignen sich ferner für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z. B. ligninsulfonsaures Natrium, 2,2'-dinaphthylmethan-6,6'-disulfonsaures Natrium, dibutylnaphthalinsulfonsaures Natrium oder auch oleoylmethyltaurinsaures Natrium enthalten. Ihre Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitan-fettsäureester oder Polyoxethylen-sorbitester.

Stäubemittel werden durch Vermahlen des Wirkstoffes mit fein verteilten, festen Stoffen, z. B. Talkum, natürlichen Tonen wie Kaolin, Bentonit, Pyrophillit odor Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z. B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 10 bis 80 Gew.-% betragen. Staubförmige Formulierungen enthalten meistens 5 bis 20 Gew.-% an Wirkstoff, versprühbare Lösungen etwa 1 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z. B. Insektiziden, Akariziden, Herbiziden, Düngemitteln, Wachstumsregulatoren oder weiteren Fungiziden sind gegebenenfalls möglich, wobei u. U. auch synergistische Wirkungssteigerungen erzielt werden können.

Im folgenden seien einige Formulierungsbeispiele angeführt:

Ein Stäubemittel wird erhalten, indem man 10 Gewichtsteile Wirkstoff und 90 Gewichtsteile Talkum als Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat stellt man her, indem man 20 Gewichtsteile Wirkstoff mit 6 Gewichtsteilen Alkylphenolpolyglykolether (z.B. ®Triton X 207 der Rohm und Haas Co.), 3 Gewichtsteilen Isotridecanolpolyglykolether (8 AeO) und 71 Gew.-T. paraffinischem Mineralöl (Siedebereich z. B. ca. 255 bis über 377 °C) mischt und in einer Reibkugelmühle auf eine Feinheit von unter 5 Mikron vermahlt.

Ein emulgierbares Konzentrat läßt sich herstellen aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

Herstellungsbeispiele

Beispiel V1 (nicht erfindungsgemäß, nach EP-A 0 068 144)
1,1-Diphenyl-2-(1,2,4-triazol-1-yl)-ethan

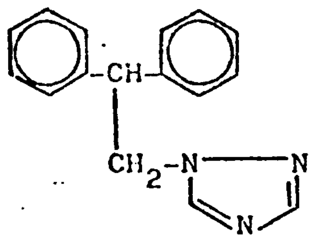

26,7 g (0,2 Mol) $AlCl_3$ gibt man portionsweise bei -10°C zu einer Lösung von 18,9 g (0,1 Mol) 1-Phenyl-2-(1,2,4-triazol-1-yl)-ethanol in 50 ml Benzol und 50 ml 1,2-Dichlorethan. Danach erwärmt man innerhalb 30 min. auf Raumtemperatur und heizt dann bis 80°C auf, bis keine Gasentwicklung mehr beobachtet wird. Man gießt auf 200 ml Eiswasser, trennt die organische Phase ab, engt ein und reibt den Rückstand mit Diisopropylether an.

Beispiele 1 bis 20

Nach der Arbeitsweise des Beispieles V1 wurden die in nachstehender Tabelle aufgeführten Verbindungen der Formel

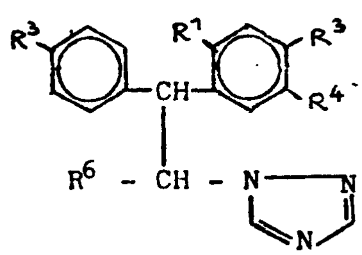

hergestellt:

| Bsp. Nr. | Ausgangsmaterialien | | | | physkal. Kenndaten |
|---|---|---|---|---|---|
| | 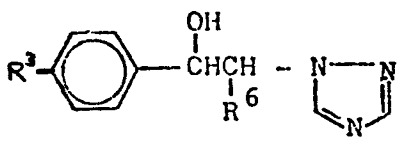 | | 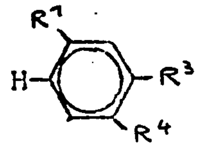 | | Kp (°C) bei Druck (mbar) bzw. Schmelzpunkt |
| | $R^6$ | $R^3$ | $R^1$ | $R^3$, $R^4$ *) | |
| 1 | $CH_3$ | H | H | H | 95-6 |
| 2 | $CH_3$ | H | H | 4-Br | 136-40 |
| 3 | $CH_3$ | H | H | 4-Cl | 180-3/0,003 |
| 4 | $CH_3$ | H | H | 4-$CH_3$ | 178-81/0,003 |
| 5 | $CH_3$ | H | H | 4-$OCH_3$ | 90 |
| 6 | $CH_3$ | H | 2-$CH_3$ | 4-$CH_3$ | 185-7/0,001 |
| 7 | $CH_3$ | H | 2-$CH_3$ | 5-$CH_3$ | 166-74/0,0008 |
| 8 | $C_2H_5$ | H | H | H | 113-8 |
| 9 | $C_2H_5$ | H | H | 4-$CH_3$ | 160-74/0,0007 |
| 10 | $C_2H_5$ | H | H | 4-$OCH_3$ | 153-6 |
| 11 | $C_2H_5$ | H | H | 4-Cl | 178-82/0,0007 |
| 12 | $C_2H_5$ | 4-Br | H | 4-Br | 215-26/0,01 |
| 13 | $C_2H_5$ | 4-$CH_3$ | H | 4-$C(CH_3)_3$ | 144-7 |
| 14 | $C_2H_5$ | 4-$CH_3$ | H | 4-$CH_3$ | 109-13 |
| 15 | $C_2H_5$ | 4-$CH_3$ | H | 4-$OCH_3$ | 202-16/0,001 |
| 16 | $C_2H_5$ | H | 2-$CH_3$ | 4-$CH_3$ | 160-8 |
| 17 | $C_2H_5$ | H | 2-$CH_3$ | 5-$CH_3$ | 121-6 |
| 18 | $C_2H_5$ | 4-$CH_3$ | 2-$CH_3$ | 4-$CH_3$ | 121-3 |
| 19 | $C_2H_5$ | 4-$CH_3$ | 2-$CH_3$ | 5-$CH_3$ | 175-8 |
| 20 | $CH_3$ | H | 2-Cl | 4-Cl | 196-98/0,01 |

*) der andere, nicht bezeichnete Rest ist jeweils H

## Biologische Beispiele

In den folgenden Beispielen stehen die Buchstaben A, B, C und D für die nachstehend genannten handelsüblichen Vergleichsmittel mit bekannten fungiziden Wirkstoffen:

A: Methyl-1-(butylcarbamoyl-2-benzimidazolcarbamat) (Benomyl)

B: N-Tridecyl-2,6-dimethyl-morpholin (Tridemorph)

C: 5-Methyl-1,2,4-triazolo-/3,4-b/-benzothiazol (Tricyclazol)

D: 5,6-Dihydro-2-methyl-1,4-oxathiin-3-carboxanilid-4,4-dioxid

## Beispiel I

Weizenpflanzen werden im 3-Blattstadium mit Konidien des Weizenmehltaus (Erysiphe graminis) stark inokuliert und in einem Gewächshaus bei 20°C und einer relativen Luftfeuchte von 90 - 95 % aufgestellt. 3 Tage nach Inokulation werden die Pflanzen mit den in Tabelle I aufgeführten Verbindungen in den Wirkstoffkonzentrationen von 500, 250, 125, 60 und 30 mg/Liter Spritzbrühe tropfnaß gespritzt. Als Vergleich wird das Vergleichsmittel B in analoger Weise eingesetzt. Nach einer Inkubationszeit von 10 Tagen werden die Pflanzen auf Befall mit Weizenmehltau untersucht. Der Befallsgrad wird ausgedrückt in % befallener Blattfläche, bezogen auf unbehandelte, infizierte Kontrollpflanzen (= 100 % Befall). Das Ergebnis ist in der Tabelle I zusammengefaßt.

0146064

Tabelle I

| Verbindung gemäß Beispiel Nr. | Mit Weizenmehltau befallene Blattfläche in % bei mg Wirkstoff/l Spritzbrühe 500 | 250 | 125 | 60 | 30 |
|---|---|---|---|---|---|
| 1 | 0 | 0 | 0 | 0-3 | 3 |
| 2 | 0 | 0 | 0 | 0 | 0 |
| 3 | 0 | 0 | 0 | 0 | 0 |
| 4 | 0 | 0 | 0 | 0 | 0 |
| 5 | 0 | 0 | 0 | 0-3 | 3 |
| 6 | 0 | 0 | 0 | 0 | 0-3 |
| 7 | 0 | 0 | 0 | 0 | 0 |
| 8 | 0 | 0 | 0 | 0 | 0 |
| 9 | 0 | 0 | 0 | 0 | 0 |
| 10 | 0 | 0 | 0 | 0 | 0 |
| 11 | 0 | 0 | 0 | 0 | 0 |
| 12 | 0 | 0 | 0 | 0 | 0 |
| 13 | 0 | 0 | 0 | 0 | 0 |
| 14 | 0 | 0 | 0 | 0 | 0 |
| 15 | 0 | 0 | 0 | 0 | 0 |
| 16 | 0 | 0 | 0 | 0 | 0 |
| 17 | 0 | 0 | 0 | 0 | 0 |
| 18 | 0 | 0 | 0 | 0 | 0 |
| 19 | 0 | 0 | 0 | 0 | 0 |
| Vergleichsmittel B | 3 Phytotoxis | 5 Phytotoxis | 10 | 15 | 25 |
| Unbehandelte, infiz. Pflanzen | | 100 | | | |

Beispiel II

Reispflanzen wurden im 4-Blattstadium mit den in Tabelle II angegebenen Verbindungen in Konzentrationen von 500, 250 und 120 mg Wirkstoff pro Liter Spritzbrühe tropfnaß gespritzt. Nach dem Abtrocknen des Spritzbelages wurden die Pflanzen mit einer Sporensuspension von Piricularia oryzae gleichmäßig besprüht und für 48 h in eine dunkel gehaltene Klimakammer mit 25°C und 100 % relativer Luftfeuchte gestellt. Anschließend wurden die Pflanzen in einem Gewächshus bei 25°C und 85 % relativer Luftfeuchte gehalten und 14 Tage nach Inokulation auf Befall mit Piricularia oryzae untersucht. Der Befallsgrad wurde ausgedrückt in % befallener Blattfläche, bezogen auf unbehandelte, infizierte Kontrollpflanzen (= 100 % Befall).

Tabelle II

| Verbindung gemäß Beispiel Nr. | % befallene Blattfläche bei mg Wirkstoff/Liter Spritzbrühe 500 | 250 | 120 |
|---|---|---|---|
| 1 | 0 | 0 | 0-3 |
| 2 | 0 | 0 | 0 |
| 3 | 0 | 0 | 0-3 |
| 5 | 0 | 0 | 0-3 |
| 6 | 0 | 0 | 0-3 |
| 7 | 0 | 0 | 0-3 |
| 15 | 0 | 0 | 0-3 |
| Vergleichsmittel C | 0 | 3 | 5 |
| Unbehandelte, infiz. Pflanzen | | 100 | |

Beispiel III

Weizenpflanzen wurden mit den in Tabelle III genannten Verbindungen in den Aufwandmengen von 500, 250, 120 und 60 mg/Liter Spritzbrühe tropfnaß behandelt. Nach dem Abtrocknen des Wirkstoffbelages wurden die Pflanzen mit Sporen des Weizenbraunrostes (Puccinia triticina) stark inokuliert und für 24 Stunden in eine Klimakammer mit 100 % rel. Luftfeuchte von 20°C gestellt. Anschließend kamen die Pflanzen in ein Gewächshaus und wurden hier 14 Tage nach Inokulation auf Befall mit Weizenbraunrost untersucht. Als Vergleichsmittel diente D.

Tabelle III

| Verbindung gemäß Beispiel Nr. | mit Weizenbraunrost befallene Blattfläche in % bei mg Wirkstoff pro Liter Spritzbrühe 500 | 250 | 120 | 60 |
|---|---|---|---|---|
| 1 | 0 | 0 | 0 | 0-3 |
| 2 | 0 | 0 | 0 | 0 |
| 3 | 0 | 0 | 0 | 0 |
| 5 | 0 | 0 | 0-3 | 3 |
| 6 | 0 | 0 | 0 | 0-3 |
| 7 | 0 | 0 | 0-3 | 3 |
| Vergleichsmittel D | 5 | 10 | 15 | 35 |
| unbehandelte, infiz. Pflanzen | | 100 | | |

Als technische Biozide zeigten einige Verbindungen ebenfalls eine gute, breit wirksame fungizide und z.T. auch bakterizide Wirkung, hier insbesondere gegen Bacillus subtilis:

Beispiel IV

Mycelstücke (Ø 0,5 cm) des Pilzes Poria monticola wurden in Petrischalen auf Nährboden (Biomalz-Agar für Pilze) im Zentrum aufgebracht; dem Agar waren zuvor im flüssigen Zustand die beanspruchten Verbindungen in den in Tabelle IV angegebenen Konzentrationen zugesetzt worden. 8 Tage nach der Beimpfung der Platten wurde der Durchmesser des Pilzmycels auf dem Agar ausgemessen und die durch die Präparate hervorgerufene Wachstumshemmung ausgedrückt in Prozent, bezogen auf die Kontrolle (= beimpfter Agar ohne Wirkstoffzusatz = 0 % Hemmung).

Tabelle IV

| Verbindungen gemäß Beispiel | Hemmung von Poria monticola in % bei mg Wirkstoff/Liter Agar | | | | |
|---|---|---|---|---|---|
| | 100 | 50 | 10 | 5 | 1 |
| 1 | 100 | 100 | 50 | 0 | |
| 2 | 100 | 100 | 80 | 50 | 0 |
| 3 | 100 | 100 | 50 | 50 | 0 |

Beispiel V

Jeweils 0,02 ml einer Sporensuspension von Ulocladium consortiale, Aureobasidium pullulans und Aspergillus niger wurden in Petrischalen auf Nährböden (Biomalz-Agar für Pilze) tropfenförmig aufgebracht; dem Agar waren zuvor im flüssigen Zustand die beanspruchten Verbindungen in den in Tabelle V angegebenen Konzentrationen zugesetzt worden. 6 Tge nach der Beimpfung der Platten wurde der Durchmesser der Pilzkolonien auf dem Agar ausgemessen und die durch die Präparate hervorgerufene Wachstumshemmung ausgedrückt in Prozent, bezogen auf die Kontrolle (= beimpfter Agar ohne Wirkstoffzusatz = 0 % Hemmung).

Tabelle V

| Verbindungen gemäß Beispiel | | Hemmung von Ulocladium consortiale, Aureobasidium pullulans und Aspergillus niger in % bei mg Wirkstoff/Liter Agar | | | |
|---|---|---|---|---|---|
| | | 100 | 50 | 10 | 5 |
| 3 | Uc | 100 | 80 | 50 | 50 |
| | Ap | 80 | 80 | 50 | 0 |
| | An | 80 | 80 | 50 | 0 |

Beispiel VI

Jeweils 0,02 ml einer Bakteriensuspension von Bacillus subtilis wurden in Petrischalen auf Nährboden (Standard - I - Nähragar für Bakterien) tropfenförmig aufgebracht; dem Agar waren zuvor in flüssigem Zustand die beanspruchten Verbindungen in den in Tabelle VI angegebenen Konzentrationen zugesetzt worden. Die mit Bakterien beimpften Platten wurden nach 4 Tagen ausgewertet; hierbei wurde die Hemmung des Wachstums im Vergleich zur Kontrolle (= beimpfter Agar ohne Wirkstoff-Zusatz = 0 % Hemmung) bonitiert.

Tabelle VI

| Verbindungen gemäß Beispiel | Hemmung von Bacillus subtilis in % bei mg Wirkstoff/Liter Agar | | | | |
|---|---|---|---|---|---|
| | 100 | 50 | 10 | 5 | 1 |
| 2 | 100 | 100 | 50 | 0 | |
| 3 | 100 | 50 | 50 | 0 | |
| 6 | 100 | 50 | 50 | 0 | |
| 9 | 100 | 50 | 50 | 0 | |
| 12 | 100 | 100 | 50 | 0 | |
| 17 | 100 | 100 | 50 | 50 | 0 |

<u>Patentansprüche</u>(für BE, CH, DE, FR, GB, IT, LI, NL und SE)

1. 1,1-Diphenyl-2-triazolyl-2-alkyl-ethane der allgemeinen Formel (I)

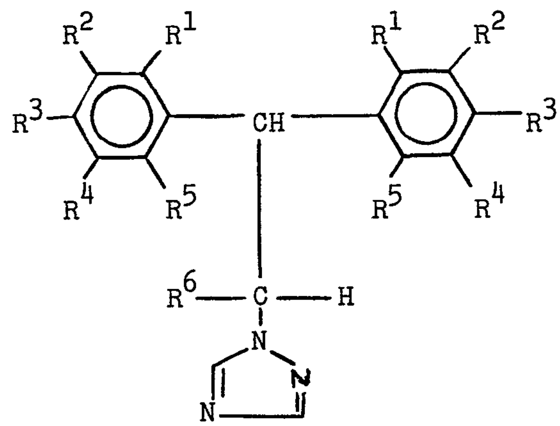

(I)

in der die Symbole folgende Bedeutung haben:

$R^1$ bis $R^5$ sind gleich oder verschieden H, Hal, $CF_3$, $(C_1-C_8)$Alkyl oder $(C_1-C_6)$Alkoxy, wobei auch die Substituenten mit gleichem Index in beiden Phenylresten jeweils verschiedene sein können, und

$R^6$ $(C_1-C_4)$Alkyl,

sowie ihre Salze, Komplexsalze und Quaternisierungsprodukte.

2. Verfahren zur Herstellung der Verbindungen der allgemeinen Formel (I) nach Anspruch 1, dadurch gekennzeichnet, daß man Verbindungen der allgemeinen Foreml (II)

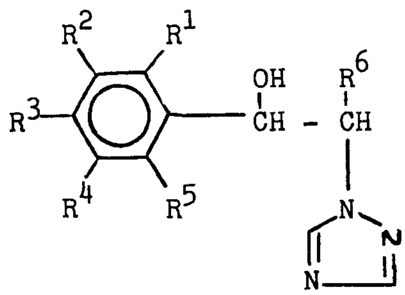

(II)

mit einem Äquivalent eines Aromaten der allgemeinen Formel (III)

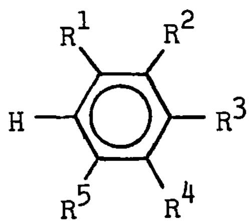

(III)

im Temperaturbereich von -15°C bis +150°C in Gegenwart eines sauren Katalysators und gegebenenfalls in Anwesenheit eines Verdünnungsmittels, einer Friedel-Crafts-Reaktion unterwirft.

3. Verfahren nach Anspruch 2, dadurch gekennzeichnet, daß der saure Katalysator Aluminiumchlorid ist.

4. Biozide Zubereitungen, die mindestens eine Verbindung nach Anspruch 1 enthalten.

5. Verwendung der Verbindungen nach Anspruch 1 als Biozide.

 

Patentansprüche (für AT)

1. Biozide Zubereitungen mit einem Gehalt an 1,1-Diphenyl-2-triazolyl-2-alkyl-ethanen der allgemeinen Formel (I)

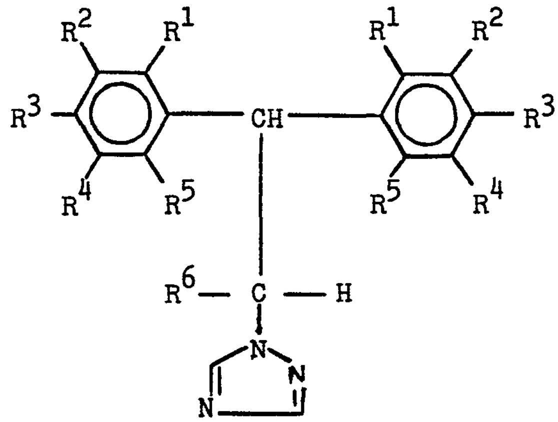

(I)

in der die Symbole folgende Bedeutung haben:

$R^1$ bis $R^5$ sind gleich oder verschieden H, Hal, $CF_3$, $(C_1-C_8)$Alkyl oder $(C_1-C_6)$Alkoxy, wobei auch die Substituenten mit gleichem Index in beiden Phenylresten jeweils verschiedene sein können, und

$R^6$ $(C_1-C_4)$Alkyl,

sowie ihren Salzen, Komplexsalzen und Quaternisierungsprodukte.

2. Verfahren zur Herstellung der Verbindungen der allgemeinen Formel (I) nach Anspruch 1, dadurch gekennzeichnet, daß man Verbindungen der allgemeinen Foreml (II)

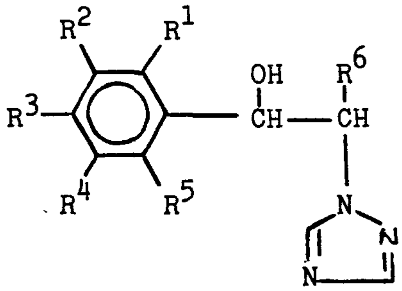

(II)

mit einem Äquivalent eines Aromaten der allgemeinen Formel (III)

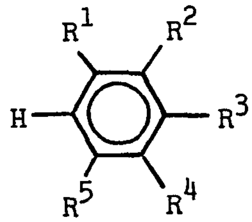

(III)

im Temperaturbereich von -15°C bis +150°C in Gegenwart eines sauren Katalysators und gegebenenfalls in Anwesenheit eines Verdünnungsmittels, einer Friedel-Crafts-Reaktion unterwirft.

3. Verfahren nach Anspruch 2, dadurch gekennzeichnet, daß der saure Katalysator Aluminiumchlorid ist.